Europäisches Patentamt

European Patent Office

Office européen des brevets

(11) Publication number: **0 069 783**
**B1**

# EUROPEAN PATENT SPECIFICATION

(45) Date of publication of patent specification: **12.11.86**

(51) Int. Cl.⁴: **G 01 N 27/12**

(21) Application number: **82900256.7**

(22) Date of filing: **13.01.82**

(86) International application number:
**PCT/JP82/00010**

(87) International publication number:
**WO 82/02598 05.08.82 Gazette 82/19**

(54) **CONTROL CIRCUIT FOR A HUMIDITY SENSOR.**

(30) Priority: **16.01.81 JP 5259/81**

(43) Date of publication of application:
**19.01.83 Bulletin 83/03**

(45) Publication of the grant of the patent:
**12.11.86 Bulletin 86/46**

(84) Designated Contracting States:
**DE FR GB SE**

(56) References cited:
**EP-A-0 009 825**
**EP-A-0 013 022**
**FR-A-2 336 777**
**JP-A-53 070 497**

**PATENTS ABSTRACTS OF JAPAN, vol. 3, no.
94, August 10, 1979, page 29 E 129**

(73) Proprietor: **Matsushita Electric Industrial Co.,
Ltd.
1006, Oaza Kadoma
Kadoma-shi Osaka-fu, 571 (JP)**

(72) Inventor: **NIWA, Takashi
3-1, Okidome 5-chome Ikaruga-cho
Ikoma-gun Nara-ken 636-01 (JP)**

(74) Representative: **Crawford, Andrew Birkby et al
A.A. THORNTON & CO. Northumberland House
303-306 High Holborn
London WC1V 7LE (GB)**

## Description

This invention relates to a control circuit for controlling the self-cleaning function of a humidity sensor. Characteristics of the control circuit are varied depending upon whether the humidity sensor is in self-cleaning mode or humidity detecting mode, by varying the circuit's resistance, thereby ensuring accurate control of the temperature of the humidity responsive region of the sensor in self-cleaning mode and an improved humidity detecting performance.

With advances in the art of sensors and microcomputers, an automated cooking appliance has been put into practical use in which a humidity sensor is provided for sensing variations in relative humidity in a heating chamber as caused by the steam generated from food and thereby monitoring the progress of cooking and then automatically completing cooking or switching an output level.

The humidity sensor, because of its very nature as a humidity detecting means, is constantly exposed to the smoke, steam, oil and so forth generated from food, with the consequence that the humidity responsive region of the sensor becomes fouled and unable to sense the humidity accurately after a long time of use. For a sensor whose humidity responsive region is made of ceramic material, that is, a so-called ceramic humidity sensor, a self-heating heater is installed on its humidity responsive surface and periodically energised to burn out such dirt and restorates its humidity responsive characteristics that is, refresh operation of the sensor. Japanese laidopen patent application No. 71686/79 discloses a cooking apparatus having a humidity sensor, the surface of which is cleaned by activating a heater situated adjacent the humidity sensor at the start and the end of the cooking period. A way to control such heating temperature depends upon the thermistor property of the sensor. Such a controller is disclosed in JP 53—70497. The controller disclosed utilises the thermistor characteristic of the sensor to decide when the sensor has reached cleaning temperature.

Accordingly it is an object of the present invention to provide a circuit arrangement which ensures more accurate measurements of humidity and refresh temperature by switching a humidity sensor control circuit during refresh operation and during humidity detection.

The present invention provides a controller circuit for a humidity detector having a resistance which varies in response to changes in temperature and changes in the humidity of the surrounding atmosphere, said controller circuit being arranged to operate in a humidity detection mode and a humidity detector cleaning mode respectively said controller circuit comprising a resistor connected in series to the detector heater means arranged to heat said detector, a power supply circuit for supplying power to said heater, means for switching said power on and off, and amplifier means connected to a point between said resistor and said detector and arranged to produce an output signal indicative of the resistance of said humidity detector, the value of said output signal for any particular resistance of the detector depending on a detector resistance vs. output voltage characteristic of said amplifier means characterised in that said controller circuit includes means arranged to change the parameters of said controller circuit whereby to change the resistance vs. output voltage characteristic depending upon whether the controller circuit is operating in humidity detection mode or humidity detector cleaning mode.

Features and advantages of the present invention will become apparent from the following description of embodiments thereof given by way of example with reference to the accompanying drawings, in which:

Figure 1 is a fragmentally front view of a microwave to which a ceramic humidity sensor is incorporated;

Figure 2 is a perspective view showing the appearance of the ceramic humidity sensor as a principal component;

Figure 3 is an enlarged cross sectional view of a humidity responsive region of the ceramic humidity sensor as shown in Figure 1;

Figure 4 is a circuit diagram of a humidity sensor control circuit;

Figure 5 is a thermistor characteristic of the humidity sensor;

Figure 6 (1) is a plot showing output voltage v. resistance characteristic of the thermistor in the circuit as illustrated in Figure 4;

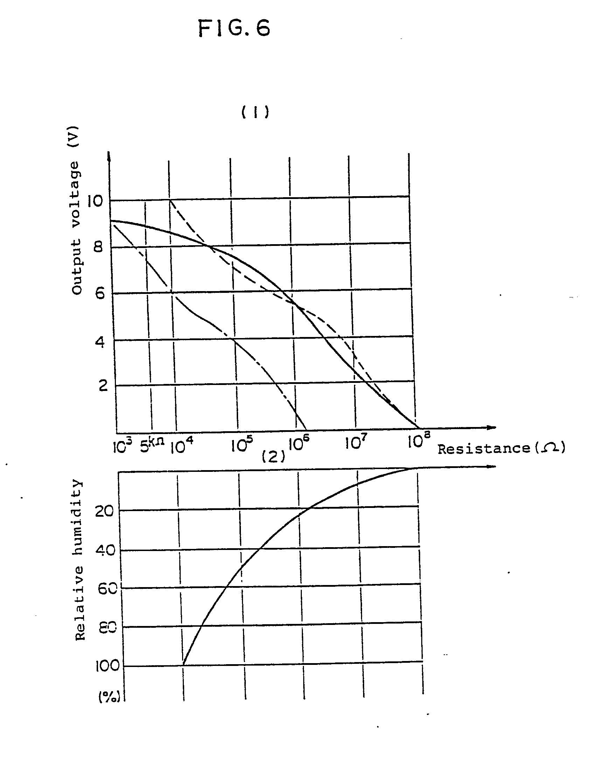

Figure 6 (2) is a plot showing relative humidity v. resistance characteristic of the humidity sensor;

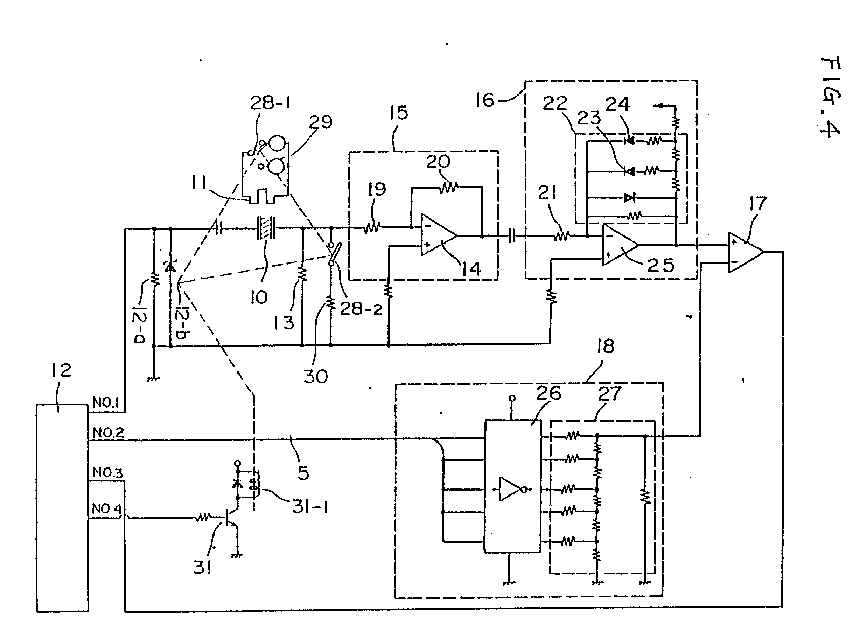

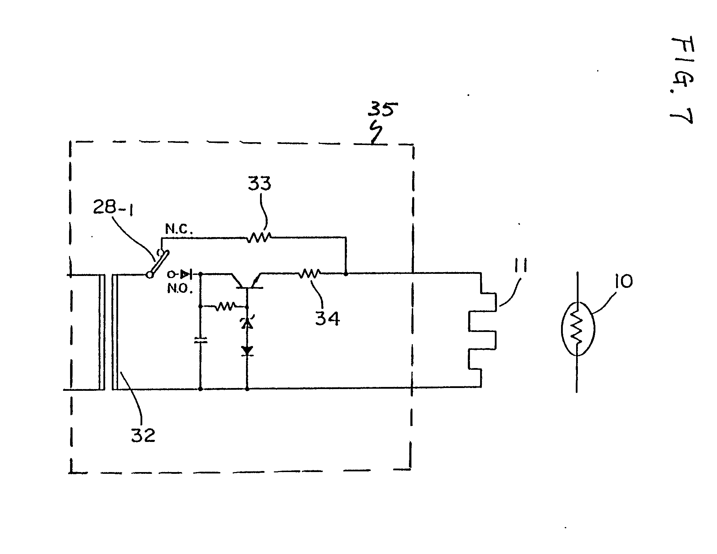

Figure 7 is a circuit diagram of a power supply circuit for a heater in Figure 4;

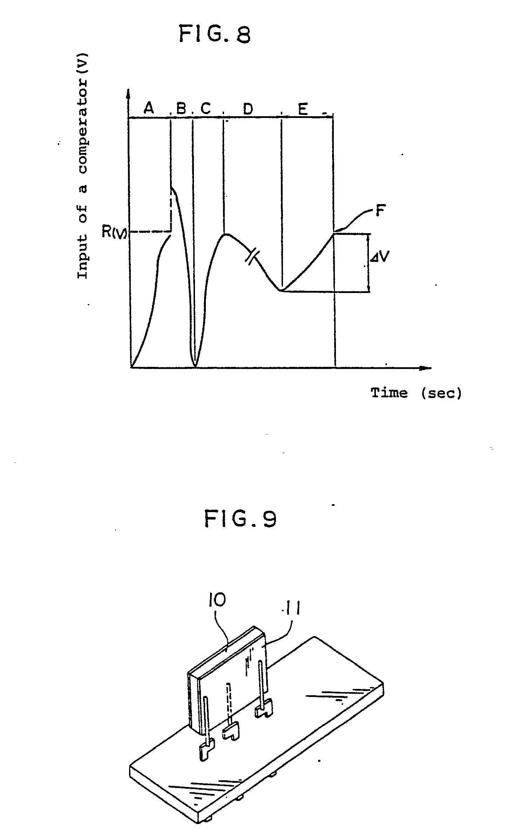

Figure 8 is a diagrammatic view of time-wise variations of an input to a comparator in Figure 4;

Figure 9 is a perspective view of a ceramic humidity sensor having a heater disposed over its humidity responsive region; and

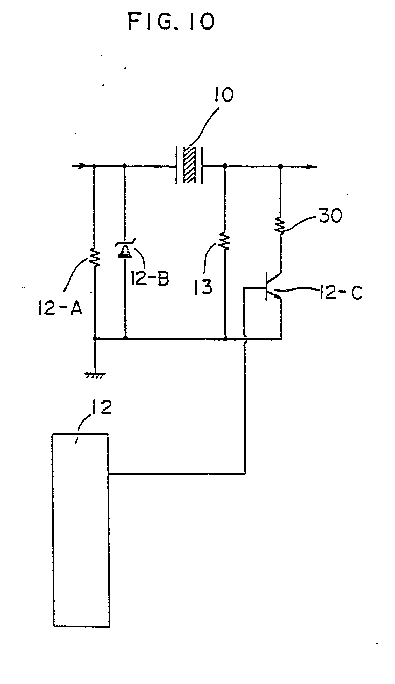

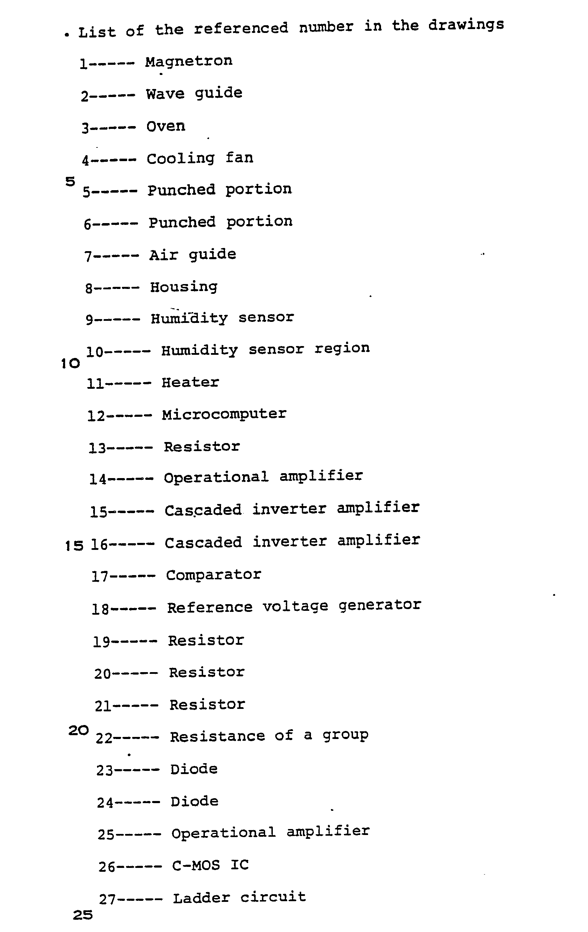

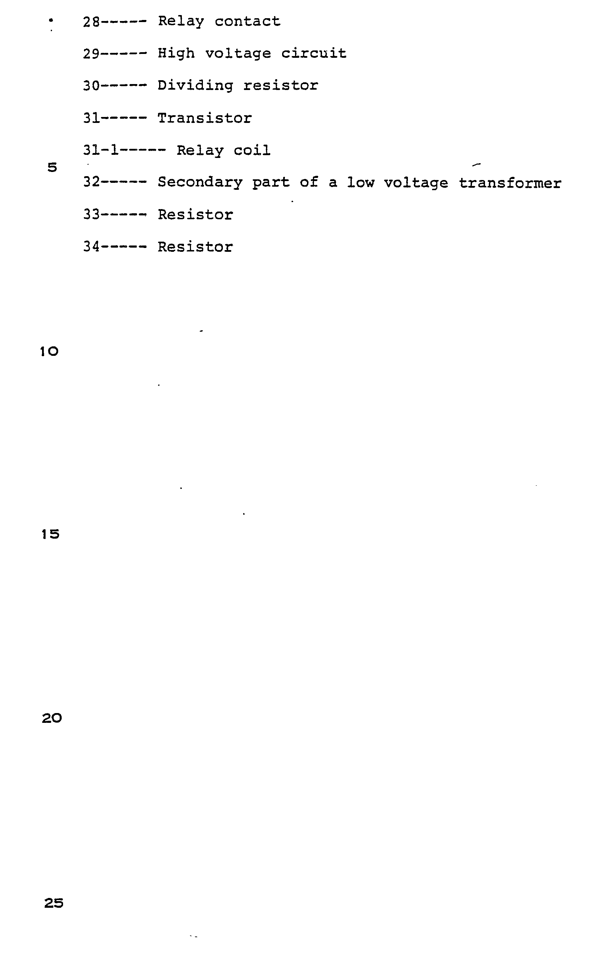

Figure 10 is a circuit diagram of a humidity sensor control circuit.

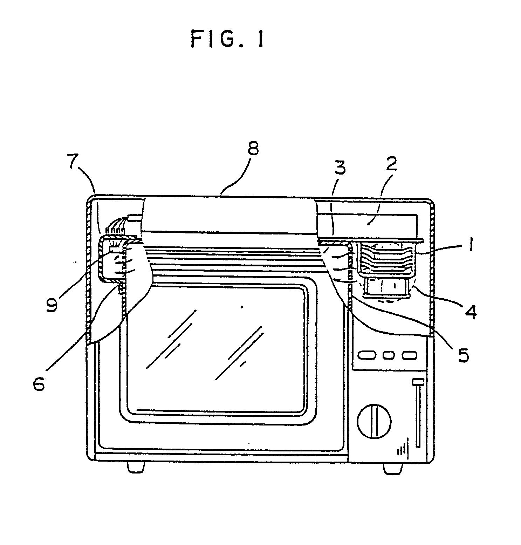

Referring to Figures 1 through 5, microwaves from a magnetron 1 are led into the interior of an oven 3 by way of a wave guide 2 and then absorbed by a food. An air flow generated by a cooling fan 4 for the magnetron 1 after cooling the magnetron 1, enters the interior of oven 3 via a punched portion 5 of a wall of the oven 3 and moves out of a housing 8 via an opposite punched portion 6 and an air guide 7. A humidity sensor 9 disposed within the air guide 7 senses the relative humidity in air outlets and in other words the relative humidity in the oven 3.

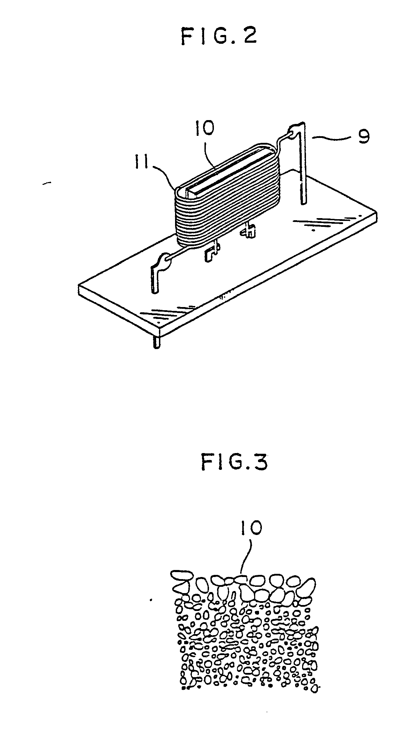

The humidity sensor 9, as shown in Figure 2, has its humidity responsive region 10 made of a metal oxide ceramic (porous sintered material consisting of $MgCr_2O_4$ and $TiO_2$) having a multiplicity of fine apertures throughout its entirety and is designed to sense the relative humidity in the oven 3 (variable with water steam generated from the food being heated) through the utilization of the phenomenon that the conductivity of the metal oxide ceramic varies as a function of humidity attached in the fine apertures.

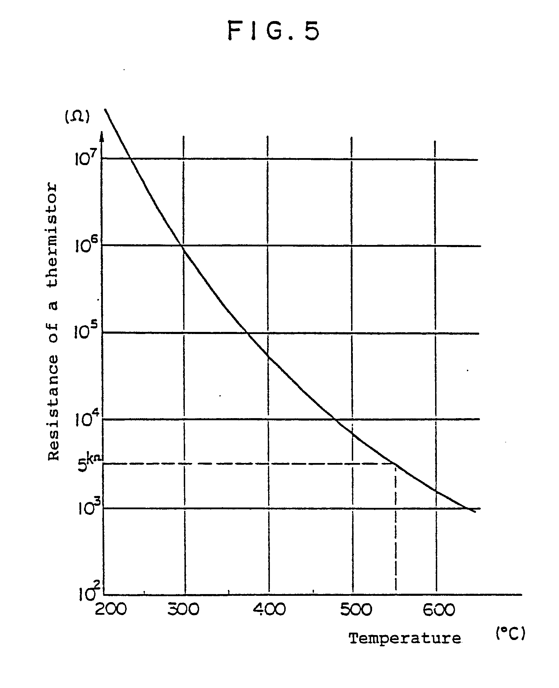

However, in the event that the humidity responsive region 10 becomes dirty with dust in the air, oil, smoke and so forth, the humidity responsive characteristics would be downgraded. An approach to overcome the problem has been suggested by which a heater 11 is installed to face against the humidity responsive region and energized to burn out such dirt and recover its humidity responsive characteristics (that is, refresh operation of the sensor). A way to control such refresh temperature relies upon the thermistor property (the resistance decreases with a temperature rise as seen from Figure 5). The characteristics of the ceramic humidity sensor, however, are that its resistance varies drastically from $10^8\Omega$ to $10^4\Omega$ due to the properties of its starting material as shown in Figure 6(2) when the relative humidity varies from 0% to 100%. To make the most of the sensor whose resistance varies drastically in the above manner, the characteristic of an amplifier necessary for converting it into a voltage of a given amplitude should be selected such that it shows a higher gain for an input signal of a small amplitude and a lower gain for that of a greater amplitude and shows a smooth variation in output voltage against $10^4$ variations in the resistance of the sensor. As indicated by the solid line in Figure 6(1), the resistance v. output voltage characteristic curve has a very gentle gradient about a refresh detection resistance of 5 K$\Omega$. In attempting to decide from the output voltage whether the humidity responsive region 10 of the humidity sensor 9 reaches the refresh temperature, the refresh temperature varies greatly due to deviations in circuit constants and voltage levels so that the refresh operation may become too much to an extent which increases excessively the temperature and destructs the sensor or become too small (an insufficient rise in temperature) to an extent which does not assure satisfactory cleaning but deteriorates the humidity responsive characteristics of the sensor.

Therefore, the object of the present invention is to provide a circuit arrangement which ensures more stable refresh operation of a sensor by differentiating resistance v. output voltage characteristic during refresh operation mode (the phantom line) from that during humidity detection mode (the dotted line) as viewed from Figure 6(1) with the aid of dividing resistors connected to an input stage of the amplifier. Details of the present invention will now be discussed with reference to Figures 4 through 8.

In Figure 4, after being stabilized through a resistor 12a and a zener diode 12b, an output voltage appearing at terminal No. 1 of a microcomputer 12 is divided through the humidity responsive region 10 of the humidity sensor and a dividing resistor 13 and applied as an input to an operational amplifier 14. After being amplified through two cascaded inverter amplifiers 15 and 16, the input voltage is applied to a + input of a comparator 17 and compared with a − input voltage from a reference voltage generator 18. The output of the comparator 17 is fed to terminal No. 3 of the microcomputer 12 so that the output voltage of a sensor circuit is derived in a digital value. The gain of the first stage inverter amplifier 15 is determined by a resistance ratio of a resistor 19 to a resistor 20, while that of the next succeeding stage inverter amplifier 16 is determined by a ratio of the resistance of a resistor 21 to the combined resistance of a group 22 of resistors. Diodes 23 and 24 are turned on and off to vary the combined resistance of the group of the feedback resistors connected to an operational amplifier 25, depending upon the amplitude of the output voltage from the operational amplifier 25, so that the output voltage shows a gentle gradient for $10^4$ variation in the resistance of the sensor. The humidity detecting property of this circuit arrangement is depicted by the dotted line in Figure 6(1). The reference voltage generator 18 is set up by a C—MOS IC 26 and a ladder circuit 27 and designed to convert 5 bit outputs from terminal No. 2 (consisting of five lines) of the microcomputer 12 into the corresponding ones of 32 (i.e. $2^5$) analog voltages.

During detection for the end of refresh operation a relay contact 28-1 is placed into a high voltage circuit side 29 to heat the self-heating heater 11. Another relay contact 28-2 is also switched on so as to place a dividing resistor 30 in parallel with the above mentioned dividing resistor 13. This results in the circuit of Figure 4 displaying a sensor resistance v. amplifier circuits output voltage characteristic such as depicted by the phantom line in Figure 6(1).

Although in the above illustrated embodiment the gradient of the resistance vs. output voltage characteristic curve of the circuit during humidity detection mode is made substantially equal to that during detection of the temperature of the humidity responsive region being heated by the heater, the present invention should not be limited thereto and all that is necessary for the present invention is the sensor resistance vs. amplifier circuits output voltage characteristic be changed depending upon whether the circuit is operating in refresh mode or in humidity detection mode.

The relay contacts 28-1 and 28-2 are activated by turning on a transistor 31 and energizing a relay coil 31-1 in response to the output of terminal No. 4 of the microcomputer 12.

Figure 7 shows power supply circuitry (35) as is clear from Figure 7, self-heating heater 11 is supplied with the output voltage of a secondary part 32 of a low voltage transformer as being divided through a resistor 33 and the self-heating heater 11, while the relay contact 28-1 is on the N.C. side. This prevents the humidity responsive region from becoming wet with dew and deteriorating its humidity response. On the other hand, while the relay contact 28-1 is on the N.O. side, the output voltage of the secondary part 32 of the low voltage transformer is DC-stabilised and divided through a resistor 34 and the self-heating heater 11 and supplied to the self-heating heater 11 to thereby heat the humidity responsive region 10. The temperature dependency of the resistance of the sensor is shown in Figure 5.

Figure 8 is a view showing the relationship between food cooking time and the voltage at the + input terminal of the comparator 17, wherein, during time slot A, the sensor resistance v. amplifier circuit output voltage characteristic is as depicted by the phantom line in Figure 6(1) i.e. the circuit is operating in refresh mode. When the amplifier circuit O/P voltage (i.e. voltage of comparator) reaches R(V) the circuit switches to display the sensor resistance v. amplifier O/P voltage characteristic depicted by the dotted line in Figure 6(1) because of the relay contact 28-1 switching to open position or the N.C. side i.e. the circuit switches to humidity detection mode. During time slot B the humidity sensor 9 recovers its humidity characteristic where the humidity is low due to a high temperature in the humidity responsive region 10 and the input to the comparator shows a sharp decline. Since the humidity sensor 9 is gradually cooled with the air from the cooling fan 4 during time slot C, the comparator input increases up to a value corresponding to the relative humidity in the atmosphere. Provided that heating goes on under these circumstances, the internal temperature of the oven 3 rises but the food has not yet generated steam so that the relative humidity in the oven 3 drops and the comparator input voltage corresponding to the relative humidity in the atmosphere therefore declines (time slot D). On the other hand, if the food starts generating steam, then the input voltage rises suddenly (time slot E). The humidity is monitored at the moment where the input increases from its minimum by ΔV (time slot F).

Figure 9 illustrates an example of the above described humidity sensor 9 having the self-heating heater 11 disposed on a surface of the humidity responsive region 10 by painting.

In Figure 10, there is illustrated another embodiment of the present invention wherein the dividing resistors are switched by a transistor and especially this transistor is switched on to place the resistor 30 into parallel relationship with the resistor 13 in response to an output from the microcomputer 12.

Industrial Applicability

As described hereinbefore, more accurate refresh detection operation is ensured even with differences in the circuit constants, by varying the value of the dividing resistors in the circuit arrangement of Figure 4 and differentiating the characteristic of the circuit arrangement during refresh mode from that during humidity detection mode, than when no difference in characteristic is found between both the operation modes. There is no fear of thermally destructing the sensor or deteriorating the humidity detection characteristic due to insufficient refreshing. Further, it is possible to use a wider range of the amplitude of the output voltage for variations in the relative humidity as seen from Figures 6(1) and 6(2) due to the distinguished refresh characteristic and to attain a higher resolution at high humidities than under other saturations. These provide advantageous features for the benefit of automatic cooking.

**Claims**

1. A controller circuit for a humidity detector (10) having a resistance which varies in response to changes in temperature and changes in the humidity of the surrounding atmosphere, said controller circuit being arranged to operate in a humidity detection mode and a humidity detector cleaning mode respectively, said controller circuit comprising a resistor (13) connected in series to the detector (10), heater means (11) arranged to heat said detector, a power supply circuit (35) for supplying power to said heater, means (28-1, 31-1) for switching said power on and off and amplifier means (15, 16) connected to a point between said resistor and said detector and arranged to produce an output signal indicative of the resistance of said humidity detector, the value of said output signal for any particular resistance of the detector depending on a detector resistance vs. output voltage characteristic of said amplifier means characterised in that said controller circuit includes means (31, 31-1, 28-2, 30) arranged to change the parameters of said controller circuit whereby to change the resistance vs. output voltage characteristic depending upon whether the controller circuit is operating in humidity detection mode or humidity detector cleaning mode.

2. A controller circuit in accordance with claim 1, wherein the gradient of the resistance vs. output voltage characteristic curve of said amplifier means during humidity detection is substantially equal to the gradient of the characteristic curve during humidity detector cleaning mode.

3. A controller circuit in accordance with any preceding claim, wherein said means arranged to change the circuit parameter includes a further resistor (30) and a relay (31-1) said relay being actuable to place said further resistor in parallel with said resistor (13).

4. A controller circuit in accordance with claims 1 or 2, wherein said means arranged to change the circuit parameters includes a semi-conductor element (12-1).

5. A controller circuit in accordance with claims 1 or 2, wherein said means arranged to change the circuit parameters includes two relay contacts (28-1, 28-2) said relay contacts also being arranged to switch the power provided from the power supply circuit (35) on and off to said heater (11).

**Patentansprüche**

1. Reglerschaltung für einen Feuchtigkeitsdetektor (10), der einen Widerstand aufweist, der sich in Abhängigkeit von Temperaturänderungen und Feuchtigkeitsänderungen der Umgebungsatmosphäre ändert, wobei die Reglerschaltung so eingerichtet ist, daß sie in einer Feuchtigkeitsermittlungsbetriebsart bzw. einer Feuchtigkeitsdetektorreinigungsbetriebsart arbeitet, wobei die Reglerschaltung einen Widerstand (13) enthält, der in Serie mit dem Detektor (10) geschaltet ist, eine Heizeinrichtung (11) zum Heizen des Detektors angeordnet ist, eine Stromversorgungsschaltung (35) zum Zuführen von Strom zu der Heizeinrichtung (28-1, 31-1) zum Ein- und Ausschalten der Stromversorgung vorgesehen ist und eine Verstärkereinrichtung (15, 16) mit einem Punkt zwischen dem Widerstand und dem Detektor verbunden und so eingerichtet ist, daß er ein Ausgangssignal erzeugt, das den Widerstand des Feuchtigkeitsdetektors angibt, wobei der Wert des Ausgangssignals für jeden speziellen Widerstand des Detektors von einer Detektorwiderstands-/Ausgangsspannungs-Charakteristik der Verstärkereinrichtung abhängt, dadurch gekennzeichnet, daß die Reglerschaltung Einrichtungen (31, 31-1, 28-2, 30) aufweist, die dazu eingerichtet sind, die Parameter der Reglerschaltung zu verändern, um die Widerstands-/Ausgangsspannungs-Charakteristik in Abhängigkeit davon zu verändern, ob die Reglerschaltung in der Feuchtigkeitsermittlungsbetriebsart oder der Feuchtigkeitsdetektorreinigungsbetriebsart arbeitet.

2. Reglerschaltung nach Anspruch 1, bei der der Gradient der Widerstands-/Ausgangsspannungs-Charakteristikkurve der Verstärkereinrichtung während der Feuchtigkeitsermittlung im wesentlichen gleich dem Gradienten der charakteristischen Kurve während der Feuchtigkeitsdetektorreinigungsbetriebsart ist.

3. Reglerschaltung nach einem der vorhergehenden Ansprüche, bei der die Einrichtung der Änderung der Schaltungsparameter einen weiteren Widerstand (30) und ein Relais (31-1) enthält, wobei das Relais dazu betätigbar ist, den weiteren Widerstand dem genannten Widerstand (13) parallelzuschalten.

4. Reglerschaltung nach Anspruch 1 oder 2, bei der die Einrichtung zur Änderung der Schaltungsparameter ein Halbleiterelement (12-1) enthält.

5. Reglerschaltung nach Anspruch 1 oder 2, bei der die Einrichtung zur Änderung der Schaltungsparameter zwei Relaiskontakte (28-1, 28-2) aufweist, welche Relaiskontakte auch dazu vorgesehen sind, die von dem Stromversorgungskreis (35) dem Heizer (11) zugeführte Spannung ein- und auszuschalten.

**Revendications**

1. Circuit de commande pour un détecteur d'humidité (10) ayant une résistance qui varie en réponse à des changements de température et des changements de l'humidité de l'atmosphère environnante, ce circuit de commande étant agencé pour fonctionner selon un mode de détection de l'humidité et selon un mode de nettoyage du détecteur d'humidité, le circuit de commande comprenant une résistance (13) connectée en série avec le détecteur (10), un élément chauffant (11) agencé pour chauffer le détecteur, un circuit d'alimentation (35) pour fournir de l'énergie à cet élément chauffant, un dispositif (28-1, 31-1) pour la mise en service et l'arrêt de cette alimentation et un dispositif amplificateur (15, 16) connecté à un point situé entre ladite résistance (13) et ledit détecteur et agencé pour produire un signal de sortie représentatif de la résistance du détecteur d'humidité, la valeur de ce signal de sortie pour toute résistance particulière du détecteur dépendant d'une caractéristique résistance du détecteur/tension de sortie du dispositif amplificateur, caractérisé en ce que le circuit de commande comporte un dispositif (31, 31-1, 28-2, 30) agencé pour changer les paramètres de ce circuit de commande, de manière à changer la caractéristique résistance/tension de sortie suivant que le circuit de commande travaille selon le mode détection de l'humidité ou selon le mode nettoyage du détecteur d'humidité.

2. Circuit de commande selon la revendication 1, dans lequel le gradient de la courbe caractéristique résistance/tension de sortie du dispositif amplificateur pendant la détection de l'humidité est sensiblement égal au gradient de la courbe caractéristique pendant le mode nettoyage du détecteur d'humidité.

3. Circuit de commande selon l'une ou l'autre des revendications précédentes, dans lequel le dispositif agencé pour changer les paramètres du circuit comporte une résistance supplémentaire (30) et un relais (31-1), ce relais pouvant être actionné pour connecter la résistance supplémentaire en parallèle avec ladite résistance (13).

4. Circuit de commande selon la revendication 1 ou 2, dans lequel le dispositif agencé pour changer les paramètres du circuit comporte un élément semi-conducteur (12-1).

5. Circuit de commande selon la revendication 1 ou 2, dans lequel le dispositif agencé pour changer les paramètres du circuit comporte deux contacts de relais (28-1, 28-2), ces contacts de relais étant également agencés pour appliquer l'énergie fournie par le circuit d'alimentation (35) à l'élément chauffant (11) et pour la couper.

# FIG. 1

# FIG.2

# FIG.3

FIG. 4

0 069 783

3

# F I G. 5

# 0 069 783

## FIG. 6

### ( I )

### ( 2 )

5

FIG. 7

# FIG. 8

Input of a comperator (V)

A B C D E F

R(V)

ΔV

Time (sec)

# FIG. 9

10  11

# FIG. 10

# 0 069 783

• List of the referenced number in the drawings

1------ Magnetron

2------ Wave guide

3------ Oven

4------ Cooling fan

5------ Punched portion

6------ Punched portion

7------ Air guide

8------ Housing

9------ Humidity sensor

10------ Humidity sensor region

11------ Heater

12------ Microcomputer

13------ Resistor

14------ Operational amplifier

15------ Cascaded inverter amplifier

16------ Cascaded inverter amplifier

17------ Comparator

18------ Reference voltage generator

19------ Resistor

20------ Resistor

21------ Resistor

22------ Resistance of a group

23------ Diode

24------ Diode

25------ Operational amplifier

26------ C-MOS IC

27------ Ladder circuit

9

**0 069 783**

28----- Relay contact

29----- High voltage circuit

30----- Dividing resistor

31----- Transistor

31-1----- Relay coil

32----- Secondary part of a low voltage transformer

33----- Resistor

34----- Resistor

10